(19) Europäisches Patentamt / European Patent Office / Office européen des brevets

(11) **EP 3 032 261 B1**

(12) **EUROPEAN PATENT SPECIFICATION**

(45) Date of publication and mention of the grant of the patent:
**23.05.2018 Bulletin 2018/21**

(51) Int Cl.:
***G01N 33/574*** (2006.01)

(21) Application number: **16153090.2**

(22) Date of filing: **17.06.2011**

(54) **MR-PRO-AMD FOR THE PREDICTION OF INCIDENT CANCER IN MALES SUBJECT BELOW THE AGE OF 57.9 YEARS**

MR-PRO-AMD FÜR DIE VORHERSAGE DER INZIDENZ EINES KARZINOMS IN MÄNLICHEN PATIENTEN UNTER 57.9 JAHRE.

MR-PRO-AMD POUR LA PRÉDICTION D'UN NOUVEAU CANCER CHEZ DES SUJETS MALES DE MOINS DE 57.9 ANS.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2010 EP 10166536**

(43) Date of publication of application:
**15.06.2016 Bulletin 2016/24**

(62) Document number(s) of the earlier application(s) in accordance with Art. 76 EPC:
**11726354.1 / 2 583 103**

(73) Proprietor: **B.R.A.H.M.S GmbH**
**16761 Hennigsdorf (DE)**

(72) Inventors:
 • **Bergmann, Andreas**
 **13645 Berlin (DE)**
 • **Struck, Joachim**
 **13465 Berlin (DE)**
 • **Melander, Olle**
 **20502 Malmö (SE)**

(74) Representative: **Hertin und Partner**
**Rechts- und Patentanwälte PartG mbB**
**Kurfürstendamm 54/55**
**10707 Berlin (DE)**

(56) References cited:
**WO-A1-2007/062676  WO-A1-2010/049178**
**WO-A2-2004/028352**

 • **OEHLER MARTIN K ET AL: "Adrenomedullin promotes formation of xenografted endometrial tumors by stimulation of autocrine growth and angiogenesis.", ONCOGENE 25 APR 2002 LNKD-PUBMED:11973640, vol. 21, no. 18, 25 April 2002 (2002-04-25), pages 2815-2821, XP002618026, ISSN: 0950-9232**
 • **GEGENHUBER ET AL: "Comparative Evaluation of B-Type Natriuretic Peptide, Mid-Regional Pro-A-type Natriuretic Peptide, Mid-Regional Pro-Adrenomedullin, and Copeptin to Predict 1-Year Mortality in Patients With Acute Destabilized Heart Failure", JOURNAL OF CARDIAL FAILURE, CHURCHILL LIVINGSTONE, NAPERVILLE, IL, US LNKD-DOI:10.1016/J.CARDFAIL.2006.09.004, vol. 13, no. 1, 3 March 2007 (2007-03-03), pages 42-49, XP005910325, ISSN: 1071-9164**

## Description

Subject of the present invention is a method of assessing the susceptibility of a male subject to acquire cancer, comprising the steps:

- Determining the level of MR-pro-ADM with SEQ ID NO: 4 or fragments thereof, having at least a lengths of 12 amino acids, in a sample obtained from said subject
- Correlating said level MR-pro-ADM with SEQ ID NO: 4 or fragments thereof with the risk of said male subject to acquire cancer, wherein said male subject does not have cancer and wherein said subject is in the age between 45.9 - 57.9 years old.

Preferably, subject of the present invention is a method for prevention monitoring (which means monitoring success of preventive measures) for a male subject, who does not have clinically manifest cancer at the time when applying this method, comprising the steps:

- Determining the level of MR-pro-ADM or fragments thereof, said fragments having at least a lengths of 12 amino acids, in a sample obtained from said subject,
- using said level of MR-pro-ADM or fragments thereof for the assessment whether or not said subject has a susceptibility to acquire cancer.

In the prior art, it has been described that pro-ADM can be used for the prediction, prognosis and therapy monitoring for other diseases than cancer. WO 2004/028352 discloses methods and uses of pro-adrenomedullin (pro-ADM) and fragments thereof for the prediction, prognosis as well as therapy monitoring, wherein age and gender can be used to improve the predictive power. Further, WO 2004/028352 teaches that ADM is secreted by many tumors. WO 2007/062676 discloses methods and uses of pro-ADM and fragment thereof for the prediction, prognosis as well as therapy monitoring, and describes peptides of at least 12 amino acids. WO 2010/049178 (2010) disclosed the use of MR-pro-ADM in a method for the diagnosis, prognosis, monitoring and therapy follow-up of disorders associated with the metabolic syndrome, a cardiovascular disease and/or insulin resistance. Gegenhuber et al. (2007) describes the use of MR-pro-ADM as a prognostic marker in patients with acute destabilized heart failure. Oehler et al. (2002) describes a pro-tumorigenic activity for ADM.

The methods of the present invention are especially valuable in the context of assessing the susceptibility of a subject to acquire cancer and are, thus, especially preferred.

Cancer is by definition a genetic disease, initiated by the activation of oncogenes and/or inactivation of suppressor genes giving rise to the typical cancer cell phenotype. Over the last decades, however, it has become evident that the progression from malignant transformation into manifest tumor disease is highly dependent on non-malignant cells of the host, perhaps most importantly through the recruitment of blood vessels, *i.e.* the "angiogenic switch". Imbalance in angiogenesis has been suggested to be of importance for development of arterial hypertension through reduced formation of arterioles and capillaries, thereby increasing the total peripheral vascular resistance. In support of this hypothesis, hypertension is a well known side effect of anti-angiogenic treatment of cancer patients. At the epidemiological level, however, hypertension has been associated with a slightly increased risk of cancer, although the cause of this relationship is unknown. Interestingly, the blood pressure sensitive and vasoactive hormones adrenomedullin, atrial natriuretic peptide and vasopressin, have been shown to have regulatory effects on angiogenesis and cancer cells in experimental models. Here, we hypothesize that host levels of adrenomedullin, atrial natriuretic peptide and/or vasopressin may have a predictive role for cancer. To address this issue, we measured stable fragments of the precursors of these hormones [midregional proadrenomedullin (MR-pro-ADM), midregional proatrial natriuretic peptide (MR-pro-ANP) and C-terminal pre-pro-Vasopressin (copeptin)] in fasting plasma in a large Swedish prospective cohort study and related baseline levels of these three biomarkers to cancer incidence during 15 years of follow-up.

Also disclosed herein is a method of assessing the susceptibility of a subject to acquire cancer and/ or assessing the risk of cancer mortality for a subject, who has not had clinically manifest cancer and/or does not have clinically manifest cancer at the time when applying this method, comprising the steps:

- Determining the level of pro-ANP or fragments thereof, and/or pro-Vasopressin or fragments thereof and/or pro-ADM or fragments thereof, any of said fragments having at least a length of 12 amino acids, in a sample obtained from said subject,
- Correlating said level of pro-ANP or fragments thereof, pro-Vasopressin or fragments thereof and/or pro-ADM or fragments thereof with the risk of said person to acquire cancer and/or with the risk of cancer mortality for said subject, respectively.

After having assessed the susceptibility and/or risk of cancer mortality the subjects may be stratified depending on the need of preventive measures.

Preferably, subject of the present invention is a method for prevention monitoring for a male subject, who does not have clinically manifest cancer at the time when applying this method, comprising the steps:

- Determining the level of MR-pro-ADM or fragments thereof, said fragments having at least a lengths of 12 amino acids, in a sample obtained from said subject,
- using said level of MR-pro-ADM or fragments thereof for the assessment whether or not said subject has a susceptibility to acquire cancer,
- or monitoring the susceptibility of said male subject to acquire cancer by repeating the determination of the level of MR-pro-ADM or fragments thereof, any of said fragments having at least a lengths of 12 amino acids.

Subject of the present invention is a method of assessing the susceptibility of a male subject to acquire cancer comprising the steps:

- Determining the level of MR-pro-ADM with SEQ ID NO:4 or fragments thereof, said fragments having at least a lengths of 12 amino acids, in a sample obtained from said subject,
- Correlating said level of MR-pro-ADM or fragments thereof with the risk of said person to acquire cancer and wherein said male subject does not have cancer and wherein said subject is in the age between 45.9 - 57.9 years old.

In a special embodiment of the above method of assessing the susceptibility of a male subject to acquire cancer said subject has not yet being diagnosed as having cancer and/or does not have cancer.

A subject not yet being diagnosed as having cancer is a subject with no prior cancer. Prior or present cancer may be confirmed by histopathology (biopsy or tissue examined after operation). This means a person which have not had cancer or does not have cancer is a person without diagnosed and/or confirmed cancer preferably by histopathology (biopsy or tissue examined after operation).

The methods of the present invention are especially preferred if the subject is a male subject. According to data collected the methods of the present invention are especially suited for a Caucasian subject, more preferred Caucasian European subject, most preferred Caucasian Northern European subject. As above stated the methods of the present invention are especially valuable in the context of assessing the susceptibility of a subject to acquire cancer and are, thus, especially preferred.

In the above method according to the invention a subject may be stratified either as having a(n enhanced) susceptibility or as haven't a(n enhanced) susceptibility to acquire cancer, either as having a(n enhanced) risk of cancer mortality or as haven't. Alternatively to such a yes/no stratification of the subjects according to risk and/ or susceptibility the stratification according to the methods of the invention may lead to more than two risk/susceptibility groups, preferably more than three risk/group with escalating risks and/or susceptibility. Certain thresholds for the biomarkers and/or mathematical combinations of biomarkers, other laboratory and clinical parameters may be defined in correlation to a certain risk/susceptibility.

Instead of stratification into certain risk groups risk stratification may be accomplished by a continuous scaling.

After having stratified a subject as having a(n enhanced) susceptibility to acquire cancer or having a(n enhanced) risk of cancer mortality several measures may be taken in order to prevent and/or delay the clinical manifestation of cancer.

These measures may encompass but are not limited to the following measures: Intensification and/or enhancing the frequency of diagnostic measures, preventive medication, change of exercise activities, change of lifestyle, change of alimentation.

Once a person has been stratified as being a subject having a(n enhanced) susceptibility to acquire cancer or having a(n enhanced) risk of cancer mortality the method of assessing the susceptibility of said subject to acquire cancer may be conducted again and/or several times in order to monitor the prevention progress.

The group of subjects not having had clinically manifest cancer and/or not having clinically manifest cancer may encompass subjects with pre-forms of cancer which are however not a clinically manifest cancer. A subject not having had clinically manifest cancer is a subject that had no prior cancer defined by clinical and histological diagnosis; a subject not having clinically manifest cancer is a subject wherein cancer is excluded according to the baseline examination *via* physical examination and questionnaire according to the Examples. In any case said subject has not yet being diagnosed as having cancer and/or does not have cancer.

Subject of the present invention is also a method for monitoring of success of preventive measures for a male subject who does not have clinically manifest cancer at the time when applying this method, comprising the steps:

- Determining the level of MR-pro-ADM or fragments thereof, said fragments having at least a lengths of 12 amino acids, in a sample obtained from said subject,

- using said level of MR-pro-ADM or fragments thereof for monitoring success of preventive measures.

In a special embodiment of the above method of the present invention said subject has not yet being diagnosed as having cancer and does not have cancer.

In an analysis shown in Examples the markers that are used according to the methods of the present invention, namely pro-ANP or fragments thereof, and/or pro-Vasopressin or fragments thereof and/or pro-ADM or fragments thereof, any of said fragments having at least a lengths of 12 amino acids predict incident cancer both, when all incident cancers are considered occurring within the next 15 years after biomarker examination, and, importantly, also, when the first four years of the 15 years follow up period are omitted from the analysis. If the methods of the present invention would be usable only for subjects having pre-forms of cancer, then it would have been expected, that prediction of incident cancer would not work for predicting cancers in years 5-15 of the follow up period. Therefore, it is clearly demonstrated that the methods of the present invention are equally predictive for subjects who might have certain pre-forms of cancer, but also for subjects not having pre-forms of cancer. In a preferred embodiment said subject is male.

In another preferred embodiment of the method according to the invention said method is used in a screening method for subjects, preferably subjects of the male population.

Said fragments are any fragments derivable from the prohormones of Adrenomedullin, Vasopressin and ANP (proAdrenomedullin, pro-Vasopressin, pro-ANP). Known fragments of pro-ADM include PAMP, MR-pro-ADM, ADM, CT-pro-ADM (Adrenotensin). Known fragments of pro-Vasopressin include Vasopressin (AVP, antidiuretic hormone, ADH), Neurophysin II, copeptin. Known fragments of pro-ANP include ANP, NT-pro-ANP, MR-pro-ANP.

The sequences of the pre-pro-hormones are as follows:

SEQ ID NO:1 (pre-pro-ADM)

```
        10         20         30         40         50         60
MKLVSVALMY LGSLAFLGAD TARLDVASEF RKKWNKWALS RGKRELRMSS SYPTGLADVK

        70         80         90        100        110        120
AGPAQTLIRP QDMKGASRSP EDSSPDAARI RVKRYRQSMN NFQGLRSFGC RFGTCTVQKL

       130        140        150        160        170        180
AHQIYQFTDK DKDNVAPRSK ISPQGYGRRR RRSLPEAGPG RTLVSSKPQA HGAPAPPSGS
```

APHFL

SEQ ID NO:2 (pre-pro-Vasopressin)

```
        10         20         30         40         50         60
MPDTMLPACF LGLLAFSSAC YFQNCPRGGK RAMSDLELRQ CLPCGPGGKG RCFGPSICCA

        70         80         90        100        110        120
DELGCFVGTA EALRCQEENY LPSPCQSGQK ACGSGGRCAA FGVCCNDESC VTEPECREGF

       130        140        150        160
```

HRRARASDRS NATQLDGPAG ALLLRLVQLA GAPEPFEPAQ PDAY

SEQ ID NO:3 (pre-pro-ANP)

```
        10          20          30          40          50          60
MSSFSTTTVS FLLLLAFQLL GQTRANPMYN AVSNADLMDF KNLLDHLEEK MPLEDEVVPP


        70          80          90         100         110         120
QVLSEPNEEA GAALSPLPEV PPWTGEVSPA QRDGGALGRG PWDSSDRSAL LKSKLRALLT


       130         140         150
```

APRSLRRSSC FGGRMDRIGA QSGLGCNSFR YRR

Additional information on the sequences:

pre-pro-ADM: http://www.uniprot.org/uniprot/P35318
pre-pro-Vasopressin: http://www.uniprot.org/uniprot/P01185
pre-pro-ANP: http://www.uniprot.org/uniprot/P01160

In an especially preferred embodiment the level of MR-pro-ADM with SEQ ID NO:4 is determined. In another especially preferred embodiment the level of copeptin with SEQ ID NO:5 is determined. In another especially preferred embodiment the level of MR-pro-ANP with SEQ ID NO:6 is determined.

SEQ ID NO:4 (MR-pro-ADM)

```
        10          20          30          40
ELRMSSSYPT GLADVKAGPA QTLIRPQDMK GASRSPEDSS PDAARIRV
```

SEQ ID NO:5 (CV-proAVP - copeptin)

```
        10          20          30
ASDRSNATQL DGPAGALLLR LVQLAGAPEP FEPAQPDAY
```

SEQ ID NO:6 (MR-pro-ANP)

```
        10          20          30
PEVPPWTGEV SPAQRDGGAL GRGPWDSSDR SALLKSKL
```

Combination of markers may give additional information. Thus, it is also a preferred embodiment according to the methods of the present invention to use the levels of pro-ANP or fragments thereof and pro-Vasopressin or fragments thereof, especially preferred is the use of MR-pro-ANP and Copeptin for the methods of the present invention.
It is also preferred embodiment according to the methods of the present invention to use the level of pro-ADM and fragments thereof and pro-ANP or fragments thereof, especially preferred is the use of MR-pro-ANP and MR-pro-ADM as a combination of marker.
In Cox proportional hazards models it was analyzed whether two markers would give independent and significant predictive information on the development of incident cancer, and thus a combination of the two would give more information than either one alone. The analysis was exemplarily done for all men.
MR-pro-ANP + Copeptin:

| _t | Haz. Ratio | Std. Err. | z | P>|z| | [95% Conf. Interval] | |
|---|---|---|---|---|---|---|
| ZLNMRANP | .8566241 | .0467314 | -2.84 | 0.005 | .7697588 | .9532918 |
| ZLNCOPEPTIN | 1.178183 | .0704958 | 2.74 | 0.006 | 1.047808 | 1.32478 |

MR-pro-ADM + MR-pro-ANP

| _t | Haz. Ratio | Std. Err. | z | P>\|z\| | [95% Conf. Interval] | |
|---|---|---|---|---|---|---|
| ZMRADM | 1.152457 | .0596426 | 2.74 | 0.006 | 1.041292 | 1.275488 |
| ZLNMRANP | .8313844 | .0465412 | -3.30 | 0.001 | .7449914 | .9277959 |

In another preferred embodiment according to the methods of the present invention the level of pro-ANP or fragments thereof, copeptin or fragments thereof and pro-ADM or fragments thereof is determined. It is especially preferred that the level MR-pro-ADM with SEQ ID NO:4, copeptin with SEQ ID NO:5 and MR-pro-ANP with SEQ ID NO:6 thereof is determined and used in the methods according to the present invention.

Further, marker may also be included in the methods of the present invention as Connective Tissue-Activating Peptide III published as a novel blood marker for early lung cancer detection (Yee et al., Connective Tissue-Activating Peptide III: A Novel Blood Biomarker for Early Lung Cancer Detection, J. Clin Onco 2009, Vol. 27:17, 2786-2792). Further marker may be neutrophil activating protein-2 (NAP-2) and haptoglobin as well as Procalcitonin and fragments thereof (EP 09011073.5) and C-Reactive Protein levels. (Zhang et al., C-Reachtive Protein Levels Are Not Associated with Increased Risk for Colorectal Cancer in Women, Annals Int. Med 2005, 142:6, 425-432). At least one of these markers may be added to the methods according to the present invention or more than of these markers selected from the group comprising Connective Tissue-Activating Peptide III, neutrophil activating protein-2, haptoglobin, Procalcitonin and fragments thereof and C-Reactive Protein.

In order to improve the accuracy of the methods of the present invention further marker/risk factors may be included into said methods. Said marker may be selected from the group comprising smoking, cancer heredity, pro-BNP or fragments thereof, Cystatin C or fragments thereof, age.

In a preferred embodiment of the invention said subject(s) are younger than 60 years. In another preferred embodiment the screening method is performed on subjects being younger than 60 years.

The inclusion of the risk factor age into the methods of the present invention is especially preferred.

In the following a specific example is given wherein certain parameters are used in order to calculate an individual's risk to get cancer. A person skilled in the art will understand that the methods of the present invention are not limited to the below specific calculation. Variations of this calculation may be performed by a person skilled in the art. *E.g.* a marker/risk factor may be added or omitted in comparison to the below calculation example.

Formula:

Based on the Cox proportional hazards model a formula can be derived, which can be used to calculate an individual's risk to get cancer. Such procedure has been used analogously in the past to develop the Framingham Risk Score for the calculation of an individual's risk to suffer from future cardiovascular events. Particular formulas may differ depending on which and how many variables have been analyzed in Cox models, and which follow-up period is considered. Principally, the formula reads:

Individual risk to get the endpoint of interest in a certain period [%] = 1 - S0^exp(xb). In this formula S0 represents the proportion of all individuals in a population, which does not get the endpoint of interest in this certain period, or more precisely: S0(T) is the survival function at T years for a subject for whom each predictor variable is equal to the average value of that variable for the entire set of subjects in the study. In this formula xb represents the sum of weighted $\beta$-coefficient of the variables from the corresponding Cox proportional hazards model, or more precisely: Xb is the sum of all risk factors minus the average of that variable in the population weighted by the regression coefficients from a Cox proportional hazards regression model.

In an example, here the formula to calculate an individual's risk to get cancer over a period of 16 years after assessment of variables has been developed for all males in the study based on Cox proportional hazards model 1.

The following variables were introduced in the model:

$$\text{smoke} = \text{current smoker (1/0)}$$

her_cancer = cancer heredity: One or more first degree relative had cancer (1/0)

$$\text{LNNTBNP} = \text{NT-BNP [pg/mL] (log-transformed)}$$

$$CYSTC = Cystatin\ C\ [mg/L]\ (log\text{-}transformed)$$

AGE = age

$$LNCOPEPTIN = Copeptin\ [pmol/L]\ (log\text{-}transformed)$$

$$MRADM = MR\text{-}pro\text{-}ADM\ [nmol/L]$$

$$LNMRANP = MR\text{-}pro\text{-}ANP\ [pmol/L]\ (log\text{-}transformed)$$

The β-coefficient of these variables from the Cox proportional hazards model were (Betas are given for 1 unit increase for continuous variables and for the condition present in dichotomous variables):

b_smoke = 0.15546
b_her_cancer = 0.09054
b_LNNTBNP = 0.00965
b_CYSTC = -0.02697
b_AGE = 0.07018
b_LNCOPEPTIN = 0.17058
b_MRADM = 0.96756
b_LNMRANP = -0.39251

xb was:

$$xb = 0.155457*(CURRENT\_SMOKER-0.274887)+0.090539*(HER\_CANCER\_0-0.444005)+0.009652*(LNNtBNP-3.860854)-0.026975*(cystC-0.795469)+0.070179*(AGE-57.530925)+0.170579*(LNCOPEPTIN-1.881057)+0.967559*(MRADM-0.451704)-0.392512*(LNMRANP-4.127731)$$

The quantitative contribution of each variable to the result is directly reflected by the absolute size of the coefficient with which the variable is multiplied with.

S0 (the proportion of all individuals in a population, which did not get cancer in the 16 year follow-up period) was 0.80.

The 16 year risk for males can be calculated as 1-0.80exp(?β(X-mean(X)).

**Examples**

**Methods:**

Study population and baseline examination procedure

The Malmö Diet and Cancer (MDC) study is a population-based, prospective epidemiologic cohort of 28449 men (born between 1923-1945) and women (born between 1923-1950) from the city of Malmö in southern Sweden who underwent baseline examinations between 1991 and 1996. From this cohort, 6103 persons were randomly selected 1991-1994 to participate in the MDC Cardiovascular Cohort (MDC-CC), which was designed to investigate the epidemiology of carotid artery disease. Fasting plasma samples were available in a total of 5543 subjects in the MDC-CC and 336 subjects had cancer prior to the baseline examination (84 males and 252 females). Subjects in the MDC-CC who had fasting plasma available, were free from prior or prevalent cancer and had data on the complete set of covariates included in model 2 (see statistical methods) were included in the dataset analyzed in the current study (4061 individuals; 1768 males and 2293 females). Prevalent, clinically manifest cancer at baseline examination was excluded by physical examination and questionnaire.

According to the studies, as outlined herein, all subjects with no prior cancer are persons which were not reported to the Swedish Cancer Register. The Swedish Cancer Register was started in 1958 and it covers 99 % of all tumors nation-wide. As 98 % have histopathological (=morphological) evidence, the register is of good quality.

Subclinical cancer: All participants underwent a careful medical history interview and standard blood samples including hemoglobin and blood cell counts, lipids and glucose. The medical history interview, which included cancer, was performed by a nurse. Abnormal blood sample results or symptoms resulted in consultation of physician at the clinic and that physician then decided whether to go for further evaluation, consult a specialist etc. Thus, the setting would be a nurse based screening with consultation of physician when indicated based on abnormal blood tests or symptoms.

Blood pressure was measured using a mercury-column sphygmomanometer after 10 minutes of rest in the supine position. Data on smoking, cancer heredity and use of antihypertensive and antidiabetic medications was ascertained from a questionnaire. Cancer heredity was defined as having at least one first degree relative diagnosed with cancer. Current smoking was defined as any cigarette smoking within the past year. Diabetes mellitus was defined as having a fasting whole blood glucose of >6.0 mmol/L, self-reported physician diagnosis of diabetes or use of antidiabetic medications. Body mass index (BMI) was defined as the weight in kilograms divided by the square of the height in meters. Myocardial infarction prior to the baseline exam was defined and retrieved as described previously.

In fasted EDTA plasma specimens were frozen immediately after collection at the MDC-CC baseline exam, we measured MR-pro-ANP, MR-pro-ADM and copeptin using immunoluminometric sandwich assays as described previously (BRAHMS, AG, Germany) (Morgenthaler et al., Measurement of Midregional Proadrenomedullin in Plasma with an Immunoluminometric Assay, Clinical Chemistry 51:10, 1823-1829 (2005)). N-terminal pro-B-type natriuretic peptide (N-BNP) was determined using the Dimension RxL automated N-BNP method (Siemens Diagnostics, Nuremberg, Germany) and cystatin C was measured using a particle-enhanced immuno-nephelometric assay (N Latex Cystatin C, Dade Behring, Deerfield, Illinois).

We measured fasting high-density lipoprotein cholesterol (HDL), insulin and triglycerides according to standard procedures at the Department of Clinical Chemistry, University Hospital Malmö and low-density lipoprotein cholesterol (LDL) was calculated according to Friedewald's formula.

All participants gave written informed consent and the study was approved by the Ethical Committee at Lund University, Lund, Sweden.

Outcomes

Cancer events were defined and subdivided according to the European Prospective Investigation on Cancer and Nutrition (EPIC) definition with the exception that cervix uteri cancer in situ was not regarded as a cancer event. Information on cancer events (both prevalent and incident events) was retrieved up until December 31st 2007 by record linkage with the Swedish Cancer Register (SCR) using a unique 10-digit civil registration number. The SCR was set up in 1958 and all malignant tumors are to be reported. Tumor site was registered according to ICD-7 and the ICD version used at diagnosis. Histopathological type was coded according to the C24 classification (REF). Approximately 99% of all tumors diagnosed at Swedish Hospitals are registered in the SCR and 98% are morphologically verified (REF). Information on total mortality and cancer mortality during follow-up was retrieved by linking the unique 10-digit civil registration number with the Swedish National Cause of Death Register (SNCDR) with ICD10 codes C00-D48 as the main cause of death (or corresponding codes in previous ICD versions) defining cancer mortality.

Statistical analyses

Comparisons in clinical characteristics between sexes were performed with t-test or Mann-Whitney test depending on normality for continuous variables and with chi-square test for dichotomous variables. We analyzed time to first event in relation to baseline biomarker levels using Cox proportional hazards models with baseline age as time scale variable. Apart from this age adjustment the three biomarkers alone and in combination were always entered into the model together with current smoking, cancer heredity, cystatin C (as marker of glomerular filtration rate) and N-BNP (as marker of sub-clinical heart failure) (model 1 covariates) unless otherwise specified in the text. The motif for adjustment for cystatin C was that the three biomarkers all are small molecules mainly cleared from plasma by glomerular filtration. The reason for entering N-BNP into the model, as a sensitive marker for subclinical heart failure and left ventricular dysfunction, was to test and adjust for any potential relationship between heart failure and cancer as all of the three biomarkers have previously been found to be elevated in heart failure.

Biomarkers with skewed distributions (MR-pro-ANP, copeptin and N-BNP) were logarithmically transformed before analysis, and the relationship between biomarker levels and incident cancer, cancer mortality and total mortality is expressed as hazard ratio per 1 standard deviation increase in the respective biomarker and in quartile analyses as hazard ratio for each quartile with the lowest quartile defined as the referent (hazard ratio 1.0) and as hazard ratio per quartile increase to obtain the P-value for trend over quartiles.

To get a summed effect estimate of the relationship between the three biomarkers and incident cancer, cancer mortality and total mortality we summed the Z-scores for the three biomarkers weighted for their respective β-coefficient from the corresponding Cox proportional hazards model (MR-pro-ADM, MR-pro-ANP and copeptin entered simultaneously together with model 1 covariates), and the weighted sum of the Z-scores was referred to as "biomarker score".

All Cox proportional hazards models which were significant after model 1 adjustment, were further adjusted in for model 2 covariates (apart from in analyses of subtypes of cancer) which included all model 1 covariates together with BMI, systolic and diastolic blood pressure, antihypertensive treatment, myocardial infarction prior to baseline, diabetes mellitus, LDL, HDL and fasting insulin.

In the Cox proportional hazards models for analyses of subtypes of cancer, the sample sizes differ from the total sample sizes and varies between the different cancer subtype analyses as subjects with other subtypes of incident cancer than the one specifically analyzed were excluded from the "control group" and the numbers of events for each subtype differs from the over-all distribution of first events (Table 2) as a first cancer subtype event was allowed to be preceded by another subtype of cancer without being censored.

In all Cox proportional hazards models, subjects were censored at the time of event, death, emigration from Sweden or at the end of follow-up. The proportionality of hazards assumption was confirmed using Schoenfeld's global test.

Crude Kaplan-Meier curves of cumulative incidence (beginning at baseline) were created for comparison of the biomarker score quartiles in analysis of incident cancer.

All analyses were performed using Stata software version 8.0 (Stata Corp) and throughout a two-sided P-value <0.05 was considered statistically significant.

**Results**

Baseline characteristics and cancer development during follow-up

The characteristics of the study population without cancer prior to and at the baseline exam are shown in Table 1. Baseline plasma concentration of N-BNP, MR-pro-ANP and MR-pro-ADM was significantly higher whereas copeptin was lower in females as compared to males. During the follow-up period [median (inter-quartile range) 14.6 (13.6-15.2) years in males and 14.8 (14.1-15.6) years in females], 366 first cancer events occurred in males and 368 in females. The complete spectrum of various subtypes of incident cancers events is shown in Table 2. In males, the most common forms of incident cancer were prostate cancer (40.4%), colorectal cancer (10.4%), pulmonary and tracheal cancer (8.5%) and urinary tract cancer (7.7%), whereas in females breast cancer (37.5%), colorectal cancer (12.0%), pulmonary and tracheal cancer (7.1%), urinary tract cancer (3.8%), corpus uteri cancer (5.2%), cervix uteri cancer (3.5%) and ovary cancer (3.8%) predominated.

Biomarkers and incident cancer

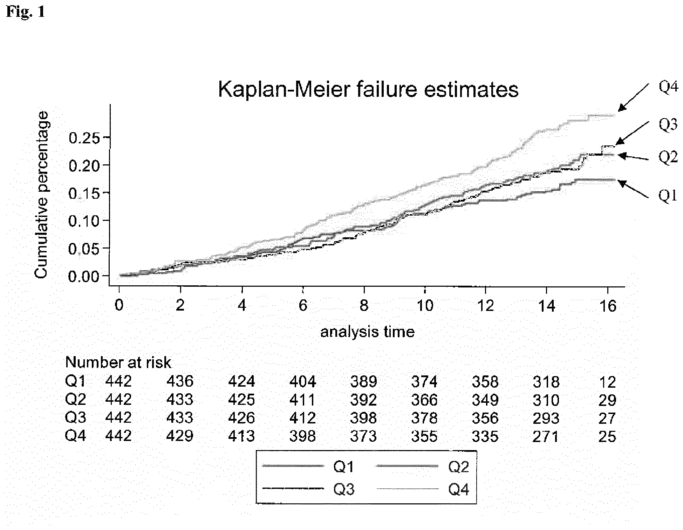

In all analyses, the proportionality of hazards assumption was met. In males, there was an independent relationship between MR-pro-ANP and copeptin, respectively, and incident cancer and a borderline significant relationship between MR-ADM and incident cancer (Table 4). As shown in analyses of quartiles, the relationship with incident cancer seemed to be graded over the distribution of MR-pro-ANP, copeptin and MR-ADM (Table 4). In contrast, N-BNP had no relationship with incident cancer with a hazard ratio (95% confidence interval) per standard deviation increase in N-BNP of 1.01 (0.90-1.13; P=0.931). When MR-pro-ANP, copeptin and MR-ADM were entered simultaneously in the model together model 1 covariates and backward elimination with a retention P-value of <0.10 was applied, the three biomarkers were all retained and significantly related to future cancer development with per standard deviation increase in biomarker level hazard ratio of 0.83 (0.75-0.93; P=0.001) for MR-pro-ANP, 1.14 (1.01-1.29; P=0.028) for copeptin and 1.12 (1.00-1.24; P=0.042) for MR-pro-ADM. To get a summed effect estimate of the relationship between the three biomarkers and incident cancer, the biomarker score (which had a negative component for MR-pro-ANP but positive for copeptin and MR-pro-ADM) was entered in the Cox proportional hazards model with model 1 covariates. The per standard deviation increase of the biomarker score hazard ratio for incident cancer was highly significant and the top versus bottom quartile of the biomarker score identified a near two-fold difference in risk of future cancer (Table 4 and Figure 1). Additional adjustment in model 2 did not change these results (not shown). In contrast, we found no evidence of association between any of the three biomarkers or the biomarker score among females (Table 6). Whether this finding is true in general, needs to be analyzed in validation studies, since there is no immediate and plausible explanation for why the association of the biomarkers work for males but not for females.

Biomarkers and cancer subtypes

To test if the relationship between the biomarkers and cancer susceptibility in males was driven by a specific subtype of cancer, we performed sub-analyses of the main cancer forms. None of the individual biomarkers were significantly related to any of the main cancer subtypes in males (prostate, colorectal, urinary tract and pulmonary/tracheal cancer) (Table 7) suggesting that the biomarkers are related to general cancer susceptibility. In line with this, the point estimates of the per standard deviation hazard ratios for the cancer subtype specific biomarker scores were all above 1.0 (between 1.13-1.25) and that of the most common form of male cancer, *i.e.* prostate cancer, was significant (P=0.041) (Table 7). In females, as in the analyses of relationship between biomarkers and general cancer susceptibility, we found no evidence of association with the main subtypes of cancer in females (Table 3).

Biomarker relationships with incident cancer in younger and older subsets of the population

Screening and any potential prevention of cancer is likely to be most meaningful in subjects with relatively long remaining life time. In addition, cardiovascular conditions affecting the levels of the studied biomarkers, such as heart failure and hypertension, as well as use of antihypertensive medications, increase steeply with age and may obscure any relationship between the biomarkers and cancer. We therefore studied the three biomarkers in relation to incident cancer separately in subjects below the median of age (range 45.9-57.9 years for males and 45.9-57.7 years for females) ("young males" and "young females") and in subjects above the median age (range 57.9-68.0 years for males and 57.7-67.9 years for females) ("old males" and "old females").

In young males (n=884 subjects who developed n=133 first incident cancer events during follow-up), the hazard ratios per standard deviation biomarker increase were significant for all three biomarkers and markedly higher than in the male population as a whole (Table 4). The hazard ratio of the biomarker score was near double as high as among all males. In quartile analyses, the risk of cancer was shown to increase gradually with the biomarker score and the difference in cancer risk between the top versus bottom quartile was more than 3-fold.

Additional adjustment in model 2 did not change these results (not shown). In older men (n=884 subjects who developed n=233 first incident cancer events during follow-up) we found no significant associations between the biomarkers and incident cancer (Table 4). Thus, each of the three studied biomarkers, and in particular the combination of the three, strongly and independently predicts cancer in the younger half of our male study population, a relationship that seems to drive the association seen in the entire group of males. Similar to the results of analyses of biomarker associations with the main subtypes of cancer in all males, the corresponding analyses in men below the median age did not reveal any specific subtype of cancer driving the biomarker association observed with general cancer susceptibility. However, as expected, the point estimates of the hazard ratios per standard deviation increase in biomarker scores were higher (ranging between 1.27-2.57) and the corresponding P-values for all of the four main subtypes of cancer were <0.10 (ranging between 0.031-0.093) (Table 7).

In females we did not detect any biomarker relationship with cancer whether the younger (n=1147 subjects who developed n=150 first incident cancer events during follow-up) or older (n=1146 subjects who developed n=218 first incident cancer events during follow-up) females were studied (Table 6).

Exclusion of early events

In order to test if the relationship between the three biomarkers and incident cancer in males is linked to mechanisms preceding cancer development or whether it is actually driven by subclinical and not yet diagnosed cancer, we excluded all cancer events diagnosed within the first four years of follow-up in analyses of all males (67 of 366 cases excluded) and young males (20 of 133 cases excluded). After these exclusions the results were similar to the results obtained without excluding early events with hazard ratio per standard deviation increase of 0.82 (0.71-0.95; P=0.009) for MR-pro-ANP, 1.16 (1.02-1.33; P=0.023) for copeptin, 1.13 (0.99-1.29; P=0.081) for MR-pro-ADM and 1.28 (1.13-1.44; P<0.001) for the biomarker score in all males. In young males the corresponding hazard ratios per standard deviation were 0.71 (0.58-0.86; P=0.001) for MR-pro-ANP, 1.24 (1.01-1.54; P=0.037) for copeptin, 1.19 (0.99-1.44; P=0.069) for MR-pro-ADM and 1.49 (1.24-1.79; P<0.001) for the biomarker score. Thus, our results indicate that decreased levels of MR-pro-ANP and increased levels of copeptin and MR-pro-ADM precede cancer development rather than being markers of already existing but non-diagnosed cancer.

Biomarkers and cancer mortality

Next, we tested whether the biomarkers are related to risk of cancer mortality (Table 8). Among 1768 males, 107 subjects died in cancer during follow-up. Whereas MR-pro-ANP and copeptin were not significantly related cancer mortality, one standard deviation increase of MR-pro-ADM was associated with a 1.25 fold increased risk of cancer mortality and males in the top quartile versus the bottom quartile of MR-pro-ADM had an almost doubled risk of cancer mortality (Table 8). The biomarker score was also related to risk of cancer mortality in males, although that association was mainly accounted for by MR-pro-ADM (Table 8). After model 2 adjustment the relationship between MR-pro-ADM and cancer mortality in males was borderline significant (P=0.085) whereas the biomarker score remained significant (P=0.031). In males, a total of 259 deaths (regardless of cause) occurred during follow-up and after model 2 adjustment, one standard deviation increase of MR-pro-ADM level significantly predicted total mortality with hazard ratio of 1.16 (1.01-1.34; P=0.035) whereas MR-pro-ANP [1.04 (0.89-1.21); P=0.655], copeptin [1.02 (0.89-1.16); P=0.790)] and the biomarker score [1.06 (0.93-1.21; P=0.404)] did not.

In the younger half of the male population, 33 subjects died in cancer during follow-up. One standard deviation increase of MR-pro-ADM was associated with a 1.55 fold increased risk of cancer mortality and the risk of dying in cancer was 2.5 fold higher in the top as compared to bottom quartile of MR-pro-ADM and 4-fold higher in the top as compared to bottom quartile of the biomarker score (Table 8) whereas MR-pro-ANP and copeptin were not individually significantly related to cancer mortality (Table 8). After model 2 adjustment MR-pro-ADM (P=0.003) and the biomarker score (P=0.002) remained significantly related to cancer mortality. Among young males a total of 66 deaths (regardless of cause) occurred during follow up and after model 2 adjustment, one standard deviation increase of MR-pro-ADM level significantly predicted total mortality with a hazard ratio of 1.35 (1.04-1.75) (P=0.023) whereas MR-pro-ANP [1.04 (0.78-1.38); P=0.792], copeptin [1.05 (0.81-1.37); P=0.702] and the biomarker score [1.17 (0.91-1.52); P=0.227] did not.

In the older half of the male population and among females (all females, young females and old females), there was no relationship between biomarker levels and cancer mortality except for in quartile analyses of MR-pro-ADM (Table 8 and Table 5) and there was no significant relationship with total mortality in any of these populations (not shown).

Table 1: Clinical characteristics of male and female study samples at baseline
Table 2: Subtypes of first incident cancer events in men and women
Table 3: Biomarkers in relation to main subtypes of incident cancer in women
Table 4: Biomarkers in relation to incident cancer in all men and in men below and above the median of age
Table 5: Biomarker levels in relation to cancer mortality in all women and in women below and above the median of age
Table 6: Biomarkers in relation to incident cancer in all women and in women below and above the median of age
Table 7: Biomarkers in relation to main subtypes of incident cancer in all men and in men below the median of age at baseline
Table 8: Biomarker levels in relation to cancer mortality in all males and in males below and above the median of age
Figure 1: Kaplan-Meier Plot demonstrating the accumulation of incident cases of clinically manifest over time per quartile of the biomarker score calculated from the measurement of MR-pro-ADM, MR-pro-ANP and copeptin at baseline (Z-scores for the three biomarkers weighted for their respective β-coefficient from the corresponding Cox proportional hazards model).

**Table 1**

| Clinical characteristics of male and female study samples at baseline | | | |
|---|---|---|---|
| Characteristic | Males (n=1768) | Females (n=2293) | P |
| Age (years) | 57.5 ± 6.0 | 57.4 ± 5.8 | 0.55 |
| Current smoking n (%) | 486 (27.5) | 558 (24.3) | 0.023 |
| Cancer heredity n (%) | 786 (44.4) | 1031 (45.0) | 0.74 |
| Cystatin C (mg/L) | 0.80 ± 0.15 | 0.76 ± 0.14 | <0.001 |
| N-BNP (pg/mL) | 46.8 (25.0-90.0) | 70.0 (42.0-122) | <0.001 |
| MR-proANP (pmol/L) | 60.6 (46.9-79.5) | 70.1 (55.1-89.3) | <0.001 |
| MR-proADM (nmol/L) | 0.45 ± 0.12 | 0.46 ± 0.13 | 0.018 |
| Copeptin (pmol/L) | 7.06 (4.58-10.5) | 4.20 (2.66-6.33) | <0.001 |
| Body mass index (m/kg$^2$) | 26.1 ± 3.4 | 25.4 ± 4.1 | <0.001 |
| Systolic blood pressure (mmHg) | 143 ± 19 | 140 ± 19 | <0.001 |
| Diastolic Blood Pressure (mmHg) | 88.6 ± 9.5 | 85.6 ± 8.9 | <0.001 |
| Antihypertensive treatment n (%) | 312 (17.6) | 369 (16.1) | 0.19 |

(continued)

| Clinical characteristics of male and female study samples at baseline | | | |
|---|---|---|---|
| Characteristic | Males (n=1768) | Females (n=2293) | P |
| Prevelant myocardial infarction n (%) | 55 (3.1) | 12 (0.5) | <0.001 |
| Diabetes Mellitus n (%) | 183 (10.3) | 127 (5.5) | <0.001 |
| Low-density lipoprotein (mmol/L) | $4.13 \pm 0.90$ | $4.19 \pm 1.04$ | 0.028 |
| High-density lipoprotein (mmol/L) | $1.21 \pm 0.30$ | $1.52 \pm 0.37$ | <0.001 |
| Insulin (mU/L) | 7.0 (5.0-10) | 6.0 (4.0-8.0) | <0.001 |

Normally distributed data are given as mean $\pm$ SD. Skewed variables are given as median (IQR).

**Table 2** Subtypes of first incident cancer events in men and women

| Cancer subtype | All men (n=1768, 366 first events) | Young men (n=884, 133 first events) | Old men (n=884, 233 first events) |
|---|---|---|---|
| Prostate, n (% of first events) | 148 (40.4) | 50 (37.6) | 98 (42.1) |
| Colorectal, n (% of first events) | 38 (10.4) | 15 (11.3) | 23 (9.9) |
| Pulmonary*, n (% of first events) | 31 (8.5) | 13 (9.8) | 18 (7.7) |
| Urinary tract**, n (% of first events) | 28 (7.7) | 5 (3.8) | 23 (9.9) |
| Skin***, n (% of first events) | 20 (5.5) | 9 (6.8) | 11 (4.7) |
| Melanoma, n (% of first events) | 14 (3.8) | 5 (3.8) | 9 (3.9) |
| Kidney, n (% of first events) | 11 (3.0) | 6 (4.5) | 5 (2.2) |
| Pancreas, n (% of first events) | 11 (3.0) | 5 (3.8) | 6 (2.6) |
| Lymphoma, n (% of first events) | 9 (2.5) | 3 (2.3) | 6 (2.6) |
| Stomach, n (% of first events) | 7 (1.9) | 2 (1.5) | 5 (2.2) |
| Nervous system, n (% of first events) | 6 (1.6) | 4 (3.0) | 2 (0.9) |
| Breast, n (% of first events) | 1 (0.3) | 0 (0.0) | 1 (0.4) |
| Unspecified, n (% of first events) | 42 (11.5) | 16 (12.3) | 26 (11.2) |
| **Cancer subtype** | **All women (n=2293, 368 first events)** | **Young women (n=1147, 150 first events)** | **Old women (n=1146, 218 first events)** |
| Breast, n (% of first events) | 138 (37.5) | 71 (47.3) | 67 (30.7) |
| Colorectal, n (% of first events) | 44 (12.0) | 9 (6.0) | 35 (16.1) |

(continued)

| Cancer subtype | All women (n=2293, 368 first events) | Young women (n=1147, 150 first events) | Old women (n=1146, 218 first events) |
|---|---|---|---|
| Pulmonary*, n (% of first events) | 26 (7.1) | 10 (6.7) | 16 (7.3) |
| Corpus uteri, n (% of first events) | 19 (5.2) | 8 (5.3) | 11 (5.1) |
| Urinary tract**, n (% of first events) | 14 (3.8) | 5 (3.3) | 9 (4.1) |
| Ovary, n (% of first events) | 14 (3.8) | 7 (4.7) | 7 (3.2) |
| Cervix uteri, n (% of first events) | 13 (3.5) | 8 (5.3) | 5 (2.3) |
| Pancreas, n (% of first events) | 12 (3.3) | 4 (2.7) | 8 (3.7) |
| Nervous system, n (% of first events) | 9 (2.5) | 4 (2.7) | 5 (2.3) |
| Skin***, n (% of first events) | 8 (2.2) | 2 (1.3) | 6 (2.8) |
| Melanoma, n (% of first events) | 8 (2.2) | 3 (2.0) | 5 (2.3) |
| Lymphoma, n (% of first events) | 7 (1.9) | 2 (1.3) | 5 (2.3) |
| Kidney, n (% of first events) | 4 (1.1) | 1 (0.7) | 3 (1.4) |
| Stomach, n (% of first events) | 4 (1.1) | 2 (1.3) | 2 (0.9) |
| Unspecified, n (% of first events) | 48 (13.0) | 14 (9.3) | 34 (15.6) |
| *Includes both pulmonary and tracheal cancer<br>**Does not include kidney cancer<br>***Does not include melanoma | | | |

Young men and women refer to the proportion of subjects of below and above median of age at baseline and old men and women refer to the proportion of subjects of above median of age at baseline.

**Table 3** Biomarkers in relation to main subtypes of incident cancer in women

| All women | | | | | | | |
|---|---|---|---|---|---|---|---|
| | | MR-proANP | | Copeptin | | MR-proADM | |
| | First events/ sample size (n)* | HR per SD (95%CI) | P | HR per SD (95%CI) | P-value | HR per SD (95%CI) | P |
| **Breast** | 142/2067 | 0.92 (0.76-1.10) | 0.351 | 0.98 (0.84-1.16) | 0.867 | 1.08 (0.89-1.31) | 0.406 |
| **Colorectal** | 47/1972 | 1.12 (0.80-1.57) | 0.520 | 0.97 (0.73-1.28) | 0.816 | 0.91 (0.66-1.26) | 0.577 |
| **Pulmonary** | 28/1953 | 1.04 (0.68-1.57) | 0.868 | 1.00 (0.70-1.43) | 0.995 | 0.64 (0.41-1.00) | 0.050 |

(continued)

| All women | | | | | | | |
|---|---|---|---|---|---|---|---|
| | | MR-proANP | | Copeptin | | MR-proADM | |
| | First events/ sample size (n)* | HR per SD (95%CI) | P | HR per SD (95%CI) | P-value | HR per SD (95%CI) | P |
| Urinary tract | 16/1941 | 1.37 (0.78-2.42) | 0.227 | 0.81 (0.58-1.15) | 0.244 | 0.87 (0.47-1.58) | 0.639 |
| Corpus uteri | 19/1944 | 1.01 (0.60-1.71) | 0.971 | 1.40 (0.72-2.72) | 0.328 | 1.08 (0.65-1.79) | 0.759 |
| Ovary | 16/1941 | 1.00 (0.56-1.79) | 1.00 | 1.24 (0.65-2.35) | 0.508 | 0.85 (0.47-1.52) | 0.578 |
| Cervix uteri | 2/1927 | n.a. | n.a. | n.a. | n.a. | n.a. | n.a. |
| *The sample size differs from the total sample of females (n=2293) and varies between the different cancer subtype analyses as subjects with other subtypes of incident cancer than the one specifically analyzed were excluded from the "control group" and the numbers of events for each subtype differs from the over-all distribution of first events (Table 2) as a first cancer subtype event was allowed to be preceded by another subtype of cancer. All analyses used age as time scale variable and were further adjusted for model 1 covariates (smoking, cancer heredity, N-BNP and cystatin C). n.a. = not analyzed due to low number of events (n=2) | | | | | | | |

**Table 4**

| Biomarkers in relation to incident cancer in all men and in men below and above the median of age | | | | | | | |
|---|---|---|---|---|---|---|---|
| All men (n=1768), number of first events (n=366) | | | | | | | |
| | HR per 1 SD | P-value | Quartile 1 | Quartile 2 | Quartile 3 | Quartile 4 | P for trend |
| MR-proANP | 0.85 (0.74-0.96) | 0.012 | 1.0 (ref) | 0.98 (0.73-1.32) | 0.85 (0.62-1.16) | 0.78 (0.56-1.10) | 0.111 |
| copeptin | 1.17 (1.04-1.32) | 0.009 | 1.0 (ref) | 1.27 (0.93-1.73) | 1.36 (1.00-1.84) | 1.45 (1.07-1.96) | 0.017 |
| MR-proADM | 1.12 (0.99-1.26) | 0.065 | 1.0 (ref) | 0.96 (0.70-1.32) | 1.05 (0.76-1.43) | 1.33 (0.96-1.82) | 0.058 |
| Biomarker score | 1.26 (1.13-1.41) | <0.001 | 1.0 (ref) | 1.32 (0.96-1.80) | 1.32 (0.96-1.82) | 1.91 (1.40-2.60) | <0.001 |
| Young men (n=884), number of first events (n=133) | | | | | | | |
| | HR per 1 SD | P-value | Quartile 1 | Quartile 2 | Quartile 3 | Quartile 4 | P for trend |
| MR-proANP | 0.75 (0.63-0.90) | 0.002 | 1.0 (ref) | 0.78 (0.50-1.23) | 0.64 (0.39-1.04) | 0.44 (0.25-0.76) | 0.003 |
| copeptin | 1.27 (1.04-1.55) | 0.017 | 1.0 (ref) | 1.34 (0.80-2.26) | 1.42 (0.85-2.39) | 1.68 (1.02-2.77) | 0.045 |
| MR-proADM | 1.23 (1.03-1.47) | 0.019 | 1.0 (ref) | 1.01 (0.60-1.68) | 1.00 (0.59-1.68) | 1.62 (1.00-2.62) | 0.055 |
| Biomarker score | 1.47 (1.23-1.75) | <0.001 | 1.0 (ref) | 1.45 (0.80-2.62) | 2.03 (1.16-3.55) | 3.35 (1.94-5.77) | <0.001 |

(continued)

| Old men (n=884), number of first events (n=233) | | | | | | | |
|---|---|---|---|---|---|---|---|
| | HR per 1 SD | P-value | Quartile 1 | Quartile 2 | Quartile 3 | Quartile 4 | P for trend |
| MR-proANP | 0.92 (0.78-1.09) | 0.346 | 1.0 (ref) | 0.95 (0.66-1.38) | 1.16 (0.80-1.67) | 0.80 (0.51-1.25) | 0.608 |
| copeptin | 1.11 (0.96-1.29) | 0.172 | 1.0 (ref) | 1.25 (0.86-1.82) | 1.19 (0.82-1.74) | 1.30 (0.89-1.90) | 0.243 |
| MR-proADM | 1.05 (0.90-1.24) | 0.525 | 1.0 (ref) | 1.30 (0.89-1.89) | 1.41 (0.96-2.05) | 1.20 (0.80-1.82) | 0.320 |
| Biomarker score | 1.14 (0.98-1.32) | 0.081 | 1.0 (ref) | 1.43 (0.97-2.11) | 1.28 (0.86-1.91) | 1.39 (0.93-2.08) | 0.209 |

All analyses used age as time scale variable and were further adjusted for model 1 covariates (smoking, cancer heredity, N-BNP and cystatin C).

Table 5 Biomarker levels in relation to cancer mortality in all women and in women below and above the median of age

| All women (n=2293), number cancer deaths (n=93) | | | | | | | |
|---|---|---|---|---|---|---|---|
| | HR per 1 SD | P-value | Quartile 1 | Quartile 2 | Quartile 3 | Quartile 4 | P for trend |
| MR-proANP | 1.00 (0.79-1.26) | 0.998 | 1.0 (ref) | 1.03 (0.55-1.95) | 1.09 (0.58-2.06) | 1.19 (0.61-2.31) | 0.578 |
| copeptin | 0.99 (0.81-1.22) | 0.945 | 1.0 (ref) | 0.87 (0.46-1.64) | 1.40 (0.80-2.43) | 0.93 (0.51-1.70) | 0.786 |
| MR-proADM | 0.96 (0.76-1.21) | 0.725 | 1.0 (ref) | 1.09 (0.58-2.07) | 1.04 (0.55-1.97) | 1.11 (0.58-2.15) | 0.797 |
| Biomarker score | 0.97 (0.78-1.20) | 0.783 | 1.0 (ref) | 1.39 (0.78-2.47) | 0.97 (0.51-1.83) | 1.00 (0.53-1.91) | 0.712 |
| Young women (n=1147), number cancer deaths (n=32) | | | | | | | |
| | HR per 1 SD | P-value | Quartile 1 | Quartile 2 | Quartile 3 | Quartile 4 | P for trend |
| MR-proANP | 0.83 (0.62-1.14) | 0.256 | 1.0 (ref) | 0.54 (0.21-1.42) | 0.67 (0.27-1.68) | 0.35 (0.11-1.10) | 0.106 |
| copeptin | 1.03 (0.73-1.45) | 0.886 | 1.0 (ref) | 0.48 (0.16-1.39) | 0.99 (0.41-2.37) | 0.59 (0.22-1.57) | 0.550 |
| MR-proADM | 0.84 (0.58-1.23) | 0.379 | 1.0 (ref) | 0.58 (0.22-1.50) | 0.46 (0.17-1.27) | 0.52 (0.19-1.42) | 0.166 |
| Biomarker score | 1.24 (0.90-1.71) | 0.181 | 1.0 (ref) | 0.52 (0.13-2.07) | 2.28 (0.86-6.04) | 1.82 (0.62-5.35) | 0.079 |
| Old women (n=1146), number cancer deaths (n=61) | | | | | | | |
| | HR per 1 SD | P-value | Quartile 1 | Quartile 2 | Quartile 3 | Quartile 4 | P for trend |
| MR-proANP | 1.17 (0.87-1.56) | 0.302 | 1.0 (ref) | 1.16 (0.49-2.76) | 2.68 (1.24-5.76) | 1.58 (0.65-3.82) | 0.101 |
| copeptin | 0.97 (0.77-1.24) | 0.831 | 1.0 (ref) | 1.50 (0.71-3.15) | 1.44 (0.69-3.02) | 1.09 (0.50-2.37) | 0.907 |

(continued)

| Old women (n=1146), number cancer deaths (n=61) | | | | | | |
|---|---|---|---|---|---|---|
| | HR per 1 SD | P-value | Quartile 1 | Quartile 2 | Quartile 3 | Quartile 4 | P for trend |
| MR-proADM | 1.05 (0.79-1.39) | 0.746 | 1.0 (ref) | 1.27 (0.57-2.79) | 1.35 (0.63-2.90) | 1.43 (0.64-3.21) | 0.391 |
| Biomarker score | 1.14 (0.86-1.52) | 0.370 | 1.0 (ref) | 1.71 (0.74-3.95) | 1.85 (0.81-4.25) | 1.99 (0.84-4.70) | 0.140 |

All analyses used age as time scale variable and were further adjusted for model 1 covariates (smoking, cancer heredity, N-BNP and cystatin C).

Young women refer to the proportion of subjects of below median of age at baseline and old women refer to the proportion of subjects of above median of age at baseline.

**Table 6** Biomarkers in relation to incident cancer in all women and in women below and above the median of age

| All women (n=2293), number of first events (n=368) | | | | | | | |
|---|---|---|---|---|---|---|---|
| | HR per 1 SD | P-value | Quartile 1 | Quartile 2 | Quartile 3 | Quartile 4 | P for trend |
| MR-proANP | 0.97 (0.87-1.10) | 0.683 | 1.0 (ref) | 1.14 (0.84-1.53) | 1.15 (0.84-1.56) | 1.08 (0.77-1.52) | 0.646 |
| copeptin | 1.00 (0.91-1.11) | 0.954 | 1.0 (ref) | 0.99 (0.74-1.32) | 1.09 (0.82-1.45) | 0.89 (0.66-1.20) | 0.617 |
| MR-proADM | 1.00 (0.86-1.12) | 0.960 | 1.0 (ref) | 1.03 (0.76-1.39) | 1.25 (0.93-1.68) | 0.89 (0.63-1.25) | 0.881 |
| Biomarker score | 1.02 (0.91-1.15) | 0.684 | 1.0 (ref) | 1.12 (0.83-1.50) | 1.03 (0.75-1.40) | 1.03 (0.74-1.44) | 0.992 |
| Young women (n=1147), number of first events (n=150) | | | | | | | |
| | HR per 1 SD | P-value | Quartile 1 | Quartile 2 | Quartile 3 | Quartile 4 | P for trend |
| MR-proANP | 0.88 (0.75-1.02) | 0.097 | 1.0 (ref) | 0.92 (0.59-1.43) | 0.99 (0.63-1.55) | 0.69 (0.41-1.17) | 0.255 |
| copeptin | 1.00 (0.85-1.17) | 0.998 | 1.0 (ref) | 0.77 (0.48-1.23) | 0.99 (0.64-1.53) | 0.91 (0.58-1.42) | 0.953 |
| MR-proADM | 0.95 (0.80-1.14) | 0.593 | 1.0 (ref) | 0.79 (0.50-1.24) | 0.71 (0.45-1.14) | 0.91 (0.57-1.46) | 0.600 |
| Biomarker score | 1.14 (0.97-1.33) | 0.106 | 1.0 (ref) | 1.22 (0.75-1.99) | 1.24 (0.75-2.04) | 1.37 (0.81-2.29) | 0.269 |
| Old women (n=1146), number of first events (n=218) | | | | | | | |
| | HR per 1 SD | P-value | Quartile 1 | Quartile 2 | Quartile 3 | Quartile 4 | P for trend |
| MR-proANP | 1.07 (0.92-1.25) | 0.381 | 1.0 (ref) | 1.29 (0.88-1.90) | 1.54 (1.03-2.27) | 1.21 (0.77-1.90) | 0.246 |
| copeptin | 1.00 (0.88-1.14) | 0.975 | 1.0 (ref) | 1.02 (0.71-1.47) | 1.02 (0.71-1.47) | 0.74 (0.49-1.10) | 0.172 |
| MR-proADM | 1.03 (0.88-1.20) | 0.711 | 1.0 (ref) | 1.16 (0.80-1.69) | 1.05 (0.71-1.54) | 1.11 (0.73-1.69) | 0.761 |

(continued)

| Old women (n=1146), number of first events (n=218) | | | | | | | |
|---|---|---|---|---|---|---|---|
| | HR per 1 SD | P-value | Quartile 1 | Quartile 2 | Quartile 3 | Quartile 4 | P for trend |
| **Biomarker score** | 0.93 (0.80-1.09) | 0.382 | 1.0 (ref) | 1.26 (0.87-1.84) | 0.91 (0.60-1.38) | 0.80 (0.51-1.24) | 0.123 |

All analyses used age as time scale variable and were further adjusted for model 1 covariates (smoking, cancer heredity, N-BNP and cystatin C).

Young women refer to the proportion of subjects of below median of age at baseline and old women refer to the proportion of subjects of above median of age at baseline.

**Table 7**

| Biomarkers in relation to main subtypes of incident cancer in all men and in men below the median of age at baseline | | | | | | | | | |
|---|---|---|---|---|---|---|---|---|---|
| **All men** | | | | | | | | | |
| | | **MR-proANP** | | **Copeptin** | | **MR-proADM** | | **Biomarker score** | |
| | First events/sample size (n)* | HR per SD (95%CI) | P | HR per SD (95%CI) | P-value | HR per SD (95%CI) | P | HR per SD (95%CI) | P |
| **Prostate** | 157/1559 | 0.87 (0.71-1.06) | 0.172 | 1.15 (0.96-1.37) | 0.131 | 1.03 (0.86-1.24) | 0.722 | 1.20 (1.01-1.43) | 0.041 |
| **Colorectal** | 42/1444 | 0.97 (0.66-1.43) | 0.872 | 1.13 (0.80-1.60) | 0.487 | 1.02 (0.71-1.45) | 0.925 | 1.13 (0.80-1.59) | 0.495 |
| **Pulmonary** | 33/1435 | 0.95 (0.62-1.47) | 0.825 | 1.22 (0.81-1.85) | 0.341 | 1.17 (0.78-1.74) | 0.445 | 1.25 (0.86-1.82) | 0.249 |
| **Urinary tract** | 33/1435 | 0.86 (0.55-1.33) | 0.493 | 1.02 (0.73-1.41) | 0.916 | 0.98 (0.67-1.45) | 0.932 | 1.15 (0.76-1.73) | 0.516 |
| **Young men** | | | | | | | | | |
| | | **MR-proANP** | | **Copeptin** | | **MR-proADM** | | **Biomarker score** | |
| | First events/sample size (n)* | HR per SD (95%CI) | P | HR per SD (95%CI) | P-value | HR per SD (95%CI) | P | HR per SD (95%CI) | P |
| **Prostate** | 52/803 | 0.82 (0.60-1.10) | 0.184 | 1.17 (0.86-1.58) | 0.317 | 1.09 (0.82-1.45) | 0.550 | 1.28 (0.96-1.70) | 0.093 |
| **Colorectal** | 16/767 | 0.67 (0.42-1.05) | 0.081 | 1.13 (0.66-1.55) | 0.658 | 0.93 (0.55-1.58) | 0.782 | 1.53 (0.93-2.49) | 0.091 |
| **Pulmonary** | 15/766 | 0.73 (0.42-1.28) | 0.268 | 1.56 (0.82-2.97) | 0.172 | 1.51 (0.93-2.45) | 0.092 | 1.81 (1.09-3.01) | 0.022 |
| **Urinary tract** | 7/758 | 0.61 (0.27-1.39) | 0.241 | 1.39 (0.56-3.41) | 0.475 | 0.52 (0.22-1.53) | 0.137 | 2.57 (1.09-6.08) | 0.031 |
| *The sample size differs from the total sample of males (n=1768) and young males (n=884) and varies between the different cancer subtype analyses as subjects with other subtypes of incident cancer than the one specifically analyzed were excluded from the "control group" and the numbers of events for each subtype differs from the over-all distribution of first events (Table 2) as a first cancer subtype event was allowed to be preceded by another subtype of cancer without being censored. All analyses used age as time scale variable and were further adjusted for model 1 covariates (smoking, cancer heredity, N-BNP and cystatin C). | | | | | | | | | |

**Table 8**

| Biomarker levels in relation to cancer mortality in all males and in males below and above the median of age | | | | | | |
|---|---|---|---|---|---|---|
| **All men (n=1768), number cancer deaths (n=107)** | | | | | | |
| | **HR per 1 SD** | **P-value** | **Quartile 1** | **Quartile 2** | **Quartile 3** | **Quartile 4** | **P for trend** |
| **MR-proANP** | 0.86 (0.68-1.10) | 0.231 | 1.0 (ref) | 1.18 (0.68-2.07) | 0.91 (0.51-1.64) | 0.80 (0.42-1.53) | 0.348 |
| **copeptin** | 1.13 (0.91-1.40) | 0.272 | 1.0 (ref) | 1.00 (0.56-1.79) | 1.23 (0.71-2.12) | 1.29 (0.75-2.22) | 0.271 |
| **MR-proADM** | 1.25 (1.00-1.56) | 0.047 | 1.0 (ref) | 1.09 (0.57-2.09) | 1.51 (0.82-2.79) | 1.91 (1.02-3.57) | 0.020 |
| **Biomarker score** | 1.28 (1.05-1.56) | 0.014 | 1.0 (ref) | 1.64 (0.89-3.02) | 2.17 (1.19-3.93) | 1.94 (1.06-3.58) | 0.025 |
| **Young men (n=884), number cancer deaths (n=33)** | | | | | | |
| | **HR per 1 SD** | **P-value** | **Quartile 1** | **Quartile 2** | **Quartile 3** | **Quartile 4** | **P for trend** |
| **MR-proANP** | 0.81 (0.55-1.19) | 0.278 | 1.0 (ref) | 1.14 (0.47-2.79) | 0.56 (0.18-1.77) | 0.78 (0.27-2.25) | 0.436 |
| **copeptin** | 1.25 (0.85-1.83) | 0.266 | 1.0 (ref) | 0.73 (0.26-2.04) | 0.85 (0.31-2.30) | 1.26 (0.52-3.05) | 0.552 |
| **MR-proADM** | 1.55 (1.11-2.15) | 0.009 | 1.0 (ref) | 0.88 (0.27-2.90) | 1.66 (0.57-4.79) | 2.53 (0.91-7.04) | 0.035 |
| **Biomarker score** | 1.65 (1.18-2.30) | 0.003 | 1.0 (ref) | 1.07 (0.27-4.32) | 3.02 (0.95-9.58) | 4.01 (1.28-12.6) | 0.003 |
| **Old men (n=884), number cancer deaths (n=74)** | | | | | | |
| | **HR per 1 SD** | **P-value** | **Quartile 1** | **Quartile 2** | **Quartile 3** | **Quartile 4** | **P for trend** |
| **MR-proANP** | 0.89 (0.66-1.19) | 0.427 | 1.0 (ref) | 0.73 (0.37-1.43) | 1.10 (0.59-2.06) | 0.52 (0.23-1.15) | 0.302 |
| **copeptin** | 1.08 (0.83-1.41) | 0.568 | 1.0 (ref) | 1.16 (0.58-2.30) | 1.58 (0.82-3.01) | 1.14 (0.56-2.30) | 0.510 |
| **MR-proADM** | 1.13 (0.85-1.51) | 0.390 | 1.0 (ref) | 1.85 (0.87-3.93) | 2.05 (0.97-4.33) | 2.51 (1.17-5.39) | 0.021 |
| **Biomarker score** | 1.18 (0.92-1.53) | 0.196 | 1.0 (ref) | 1.57 (0.74-3.32 | 2.66 (1.31-5.42) | 1.63 (0.74-3.59) | 0.109 |

All analyses used age as time scale variable and were further adjusted for model 1 covariates (smoking, cancer heredity, N-BNP and cystatin C).

## Claims

1.  A method of assessing the susceptibility of a male subject to acquire cancer, comprising the steps:

    - Determining the level of MR-pro-ADM with SEQ ID NO: 4 or fragments thereof, said fragments having at least a lengths of 12 amino acids, in a sample obtained from said subject,
    - Correlating said level of MR-pro-ADM with SEQ ID NO: 4 or fragments thereof with the risk of said male subject to acquire cancer, and wherein said male subject does not have cancer and

    wherein said subject is in the age between 45.9 - 57.9 years old.

2.  A method according to claim 1 used for monitoring of success of preventive measures for a male subject, who does not have clinically manifest cancer at the time when applying this method, comprising the steps:

    - Determining the level of MR-pro-ADM or fragments thereof, said fragments having at least a lengths of 12 amino acids, in a sample obtained from said subject,
    - using said level of MR-pro-ADM or fragments thereof for the assessment whether or not said male subject has a susceptibility to acquire cancer,
    - or monitoring the susceptibility of said male subject to acquire cancer by repeating the determination of the level of MR-pro-ADM or fragments thereof, any of said fragments having at least a lengths of 12 amino acids.

3.  A method according to claim 2 wherein once a male person has been assessed as being a subject having a(n enhanced) susceptibility to acquire cancer the method of assessing the susceptibility of said male subject to acquire cancer according to claims 1 or 2 may be conducted again and / or several times in order to monitor the prevention progress.

4. A method according to any of claims 1 to 3, wherein this method is used in a screening method for male subjects.

5. A method according to any of the claims 1 to 4, wherein the level of MR-pro-AN with SEQ ID NO:6 and copeptin with SEQ ID NO:5 are determined and correlated with the risk of said male person to acquire cancer.

6. A method according to any of the claims 1 to 5, wherein the male subject(s) age is included in the determination of the risk to acquire cancer.

7. A method according to any of the claims 1 to 6, wherein at least one of the following parameters is included in the determination of the risk to acquire cancer: smoking habits (1/0), cancer heredity, cancer of one or more first degree relative cancer.

**Patentansprüche**

1. Verfahren zur Ermittlung der Anfälligkeit eines männlichen Patienten an Krebs zu erkranken, das die folgenden Schritte umfasst:

   - Bestimmen des Pegels von MR-pro-ADM mit SEQ ID NO: 4 oder Fragmenten davon, wobei die Fragmente mindestens eine Länge von 12 Aminosäuren aufweisen, in einer Probe, die von dem Patienten erhalten wurde,
   - Korrelieren des Pegels von MR-pro-ADM mit SEQ ID NO: 4 oder Fragmenten davon mit dem Risiko, dass der männliche Patient an Krebs erkrankt, und wobei der männliche Patient nicht an Krebs leidet und

   wobei der Patient im Alter von zwischen 45,9-57,9 Jahren ist.

2. Verfahren nach Anspruch 1, das zum Überwachen von Erfolg von Präventivmaßnahmen für einen männlichen Patienten verwendet wird, der zum Zeitpunkt, an dem das Verfahren angewendet wird, nicht an klinisch manifestem Krebs leidet, das die folgenden Schritte umfasst:

   - Bestimmen des Pegels von MR-pro-ADM oder Fragmenten davon, wobei die Fragmente mindestens eine Länge von 12 Aminosäuren aufweisen, in einer Probe, die von dem Patienten erhalten wurde,
   - Verwenden des Pegels von MR-pro-ADM oder Fragmenten davon für die Beurteilung, ob ein männlicher Patient eine Anfälligkeit an Krebs zu erkranken aufweist oder nicht,
   - oder Überwachen der Anfälligkeit des männlichen Subjekts an Krebs zu erkranken durch Wiederholen der Bestimmung des Pegels von MR-pro-ADM oder Fragmenten davon, wobei ein beliebiges der Fragmente mindestens eine Länge von 12 Aminosäuren aufweist.

3. Verfahren nach Anspruch 2, wobei, sobald eine männliche Person als ein Patient ermittelt wurde, der eine (erhöhte) Anfälligkeit an Krebs zu erkranken aufweist, das Verfahren des Ermittelns der Anfälligkeit des männlichen Patienten an Krebs zu erkranken nach Anspruch 1 oder 2 erneut und/oder mehrere Male durchgeführt werden kann, um den Präventionsfortschritt zu überwachen.

4. Verfahren nach einem der Ansprüche 1 bis 3, wobei das Verfahren bei einem Screening-Verfahren für männliche Patienten verwendet wird.

5. Verfahren nach einem der Ansprüche 1 bis 4, wobei die Pegel von MR-pro-AN mit SEQ ID NO: 6 und Copeptin mit SEQ ID NO: 5 bestimmt werden und mit dem Risiko korreliert werden, dass die männliche Person an Krebs erkrankt.

6. Verfahren nach einem der Ansprüche 1 bis 5, wobei das Alter des/der männlichen Patienten in der Bestimmung des Risikos an Krebs zu erkranken beinhaltet ist.

7. Verfahren nach einem der Ansprüche 1 bis 6, wobei mindestens einer der folgenden Parameter in der Bestimmung des Risikos an Krebs zu erkranken beinhaltet ist: Rauchgewohnheiten (1/0), Krebserblichkeit, Krebs aus einer oder mehreren Krebserkrankungen von Familienmitgliedern ersten Grades.

**Revendications**

1. Procédé d'évaluation de la susceptibilité d'un sujet de sexe masculin à développer un cancer, comprenant les étapes de :

   - détermination du niveau de MR-pro-ADM avec SEQ ID NO : 4 ou des fragments de celle-ci, lesdits fragments ayant une longueur d'au moins 12 acides aminés, dans un échantillon prélevé sur ledit sujet,
   - corrélation dudit niveau de MR-pro-ADM avec SEQ ID NO : 4 ou des fragments de celle-ci au risque pour ledit sujet de sexe masculin de développer un cancer, et dans lequel ledit sujet de sexe masculin n'a pas de cancer et

   dans lequel ledit sujet est âgé de 45,9 à 57,9 ans.

2. Procédé selon la revendication 1, utilisé pour surveiller le succès de mesure préventives pour un sujet de sexe masculin, qui n'a pas de cancer cliniquement manifeste au moment d'appliquer ce procédé, comprenant les étapes de :

   - détermination du niveau de MR-pro-ADM ou des fragments de celle-ci, lesdits fragments ayant une longueur d'au moins 12 acides aminés, dans un échantillon prélevé sur ledit sujet,
   - utilisation dudit niveau de MR-pro-ADM ou des fragments de celle-ci pour l'évaluation de la susceptibilité ou non dudit sujet de sexe masculin à développer un cancer,
   - ou surveillance de la susceptibilité dudit sujet de sexe masculin à développer un cancer en répétant la détermination du niveau de MR-pro-ADM ou des fragments celle-ci, chacun desdits fragments ayant une longueur d'au moins 12 acides aminés.

3. Procédé selon la revendication 2, dans lequel dès lors qu'un individu de sexe masculin a été évalué comme étant un sujet présentant une susceptibilité (accrue) à développer un cancer, le procédé d'évaluation de la susceptibilité dudit sujet de sexe masculin à développer un cancer selon les revendications 1 ou 2 peut être réalisé de nouveau et/ou plusieurs fois de manière à surveiller l'évolution de la prévention.

4. Procédé selon l'une quelconque des revendications 1 à 3, dans lequel ce procédé est utilisé dans un procédé de dépistage pour les sujets de sexe masculin.

5. Procédé selon l'une quelconque des revendications 1 à 4, dans lequel les niveaux de MR-pro-AN avec SEQ ID NO : 6 et de copeptine avec SEQ ID NO : 5 sont déterminés et corrélés au risque pour ledit individu de sexe masculin de développer un cancer.

6. Procédé selon l'une quelconque des revendications 1 à 5, dans lequel l'âge du ou des sujets de sexe masculin est inclus dans la détermination du risque de développer un cancer.

7. Procédé selon l'une quelconque des revendications 1 à 6, dans lequel au moins l'un des paramètres suivants est inclus dans la détermination du risque de développer un cancer : habitudes tabagiques (1/0), hérédité du cancer, cancer d'un ou de plusieurs parents au premier degré.

**Fig. 1**

Kaplan-Meier failure estimates

| Number at risk | | | | | | | | | |
|---|---|---|---|---|---|---|---|---|---|
| Q1 | 442 | 436 | 424 | 404 | 389 | 374 | 358 | 318 | 12 |
| Q2 | 442 | 433 | 425 | 411 | 392 | 366 | 349 | 310 | 29 |
| Q3 | 442 | 433 | 426 | 412 | 398 | 378 | 356 | 293 | 27 |
| Q4 | 442 | 429 | 413 | 398 | 373 | 355 | 335 | 271 | 25 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004028352 A
- WO 2007062676 A
- WO 2010049178 A
- WO 2010 A
- EP 09011073 A
- EP 11726354 A

**Non-patent literature cited in the description**

- **YEE et al.** Connective Tissue-Activating Peptide III: A Novel Blood Biomarker for Early Lung Cancer Detection. *J. Clin Onco,* 2009, vol. 27 (17), 2786-2792
- **ZHANG et al.** C-Reachtive Protein Levels Are Not Associated with Increased Risk for Colorectal Cancer in Women. *Annals Int. Med,* 2005, vol. 142 (6), 425-432
- **MORGENTHALER et al.** Measurement of Midregional Proadrenomedullin in Plasma with an Immunoluminometric Assay. *Clinical Chemistry,* 2005, vol. 51 (10), 1823-1829

SEQUENCE LISTING

<110> B.R.A.H.M.S GmbH

<120> Biomarkers for the prediction of incident cancer

<130> B75001EP-A

<150> EP11726354.1
<151> 2011-06-17

<160> 6

<170> PatentIn version 3.5

<210> 1
<211> 185
<212> PRT
<213> Homo sapiens

<400> 1

```
Met Lys Leu Val Ser Val Ala Leu Met Tyr Leu Gly Ser Leu Ala Phe
1               5                   10                  15

Leu Gly Ala Asp Thr Ala Arg Leu Asp Val Ala Ser Glu Phe Arg Lys
                20                  25                  30

Lys Trp Asn Lys Trp Ala Leu Ser Arg Gly Lys Arg Glu Leu Arg Met
                35                  40                  45

Ser Ser Ser Tyr Pro Thr Gly Leu Ala Asp Val Lys Ala Gly Pro Ala
        50                  55                  60

Gln Thr Leu Ile Arg Pro Gln Asp Met Lys Gly Ala Ser Arg Ser Pro
65                  70                  75                  80

Glu Asp Ser Ser Pro Asp Ala Ala Arg Ile Arg Val Lys Arg Tyr Arg
                85                  90                  95

Gln Ser Met Asn Asn Phe Gln Gly Leu Arg Ser Phe Gly Cys Arg Phe
                100                 105                 110

Gly Thr Cys Thr Val Gln Lys Leu Ala His Gln Ile Tyr Gln Phe Thr
            115                 120                 125

Asp Lys Asp Lys Asp Asn Val Ala Pro Arg Ser Lys Ile Ser Pro Gln
        130                 135                 140

Gly Tyr Gly Arg Arg Arg Arg Arg Ser Leu Pro Glu Ala Gly Pro Gly
145                 150                 155                 160

Arg Thr Leu Val Ser Ser Lys Pro Gln Ala His Gly Ala Pro Ala Pro
```

EP 3 032 261 B1

165 170 175

Pro Ser Gly Ser Ala Pro His Phe Leu
180 185

<210> 2
<211> 164
<212> PRT
<213> Homo sapiens

<400> 2

Met Pro Asp Thr Met Leu Pro Ala Cys Phe Leu Gly Leu Leu Ala Phe
1 5 10 15

Ser Ser Ala Cys Tyr Phe Gln Asn Cys Pro Arg Gly Gly Lys Arg Ala
20 25 30

Met Ser Asp Leu Glu Leu Arg Gln Cys Leu Pro Cys Gly Pro Gly Gly
35 40 45

Lys Gly Arg Cys Phe Gly Pro Ser Ile Cys Cys Ala Asp Glu Leu Gly
50 55 60

Cys Phe Val Gly Thr Ala Glu Ala Leu Arg Cys Gln Glu Glu Asn Tyr
65 70 75 80

Leu Pro Ser Pro Cys Gln Ser Gly Gln Lys Ala Cys Gly Ser Gly Gly
85 90 95

Arg Cys Ala Ala Phe Gly Val Cys Cys Asn Asp Glu Ser Cys Val Thr
100 105 110

Glu Pro Glu Cys Arg Glu Gly Phe His Arg Arg Ala Arg Ala Ser Asp
115 120 125

Arg Ser Asn Ala Thr Gln Leu Asp Gly Pro Ala Gly Ala Leu Leu Leu
130 135 140

Arg Leu Val Gln Leu Ala Gly Ala Pro Glu Pro Phe Glu Pro Ala Gln
145 150 155 160

Pro Asp Ala Tyr

<210> 3
<211> 153
<212> PRT
<213> Homo sapiens

21

<400> 3

```
Met Ser Ser Phe Ser Thr Thr Thr Val Ser Phe Leu Leu Leu Leu Ala
1               5                   10                  15

Phe Gln Leu Leu Gly Gln Thr Arg Ala Asn Pro Met Tyr Asn Ala Val
            20                  25                  30

Ser Asn Ala Asp Leu Met Asp Phe Lys Asn Leu Leu Asp His Leu Glu
            35                  40                  45

Glu Lys Met Pro Leu Glu Asp Glu Val Val Pro Pro Gln Val Leu Ser
        50                  55                  60

Glu Pro Asn Glu Glu Ala Gly Ala Ala Leu Ser Pro Leu Pro Glu Val
65                  70                  75                  80

Pro Pro Trp Thr Gly Glu Val Ser Pro Ala Gln Arg Asp Gly Gly Ala
                85                  90                  95

Leu Gly Arg Gly Pro Trp Asp Ser Ser Asp Arg Ser Ala Leu Leu Lys
            100                 105                 110

Ser Lys Leu Arg Ala Leu Leu Thr Ala Pro Arg Ser Leu Arg Arg Ser
            115                 120                 125

Ser Cys Phe Gly Gly Arg Met Asp Arg Ile Gly Ala Gln Ser Gly Leu
        130                 135                 140

Gly Cys Asn Ser Phe Arg Tyr Arg Arg
145                 150
```

<210> 4
<211> 48
<212> PRT
<213> Homo sapiens

<400> 4

```
Glu Leu Arg Met Ser Ser Ser Tyr Pro Thr Gly Leu Ala Asp Val Lys
1               5                   10                  15

Ala Gly Pro Ala Gln Thr Leu Ile Arg Pro Gln Asp Met Lys Gly Ala
            20                  25                  30

Ser Arg Ser Pro Glu Asp Ser Ser Pro Asp Ala Ala Arg Ile Arg Val
            35                  40                  45
```

<210> 5

<211> 39
<212> PRT
<213> Homo sapiens

<400> 5

```
Ala Ser Asp Arg Ser Asn Ala Thr Gln Leu Asp Gly Pro Ala Gly Ala
1               5               10              15

Leu Leu Leu Arg Leu Val Gln Leu Ala Gly Ala Pro Glu Pro Phe Glu
                20              25              30

Pro Ala Gln Pro Asp Ala Tyr
                35
```

<210> 6
<211> 38
<212> PRT
<213> Homo sapiens

<400> 6

```
Pro Glu Val Pro Pro Trp Thr Gly Glu Val Ser Pro Ala Gln Arg Asp
1               5               10              15

Gly Gly Ala Leu Gly Arg Gly Pro Trp Asp Ser Ser Asp Arg Ser Ala
                20              25              30

Leu Leu Lys Ser Lys Leu
                35
```